# EUROPEAN PATENT APPLICATION

(11) **EP 4 785 931 A1**
(43) Date of publication of application: **05.08.2026**
(21) Application number: 24870904.0
(22) Date of filing: 26.09.2024
(51) Int. Cl.: A61K 9/20, A61K 9/48, A61K 9/16, A61K 31/57, A61K 47/38, A61K 47/26, A61K 47/04, A61P 25/00, A61P 25/20, A61P 25/28, A61P 25/08, A61P 25/24

(54) **PHARMACEUTICAL COMPOSITION COMPRISING ALLOPREGNANOLONE DERIVATIVE AND USE THEREOF**

(30) Priority: 27.09.2023 CN 202311263378
(71) Applicant: Nanjing Minova Pharmaceutical Co., Ltd., Nanjing, Jiangsu 210046 (CN)
(72) Inventor: LIU, Fei, Nanjing, Jiangsu 210046 (CN); JIANG, Weihua, Nanjing, Jiangsu 210046 (CN); XIA, Liye, Nanjing, Jiangsu 210046 (CN); ZHANG, Cuixia, Nanjing, Jiangsu 210046 (CN); WANG, Lulu, Nanjing, Jiangsu 210046 (CN)
(74) Representative: Gille Hrabal Partnerschaftsgesellschaft mbB Patentanwälte
(86) International application number: PCT/CN2024/121558
(87) International publication number: WO 2025/067365

(57) **Abstract**

Provided are a pharmaceutical composition comprising an allopregnanolone derivative and the use thereof. The pharmaceutical composition comprises the following components in percentage by weight: 30% to 70% of allopregnanolone derivative, 15% to 62% of filler, 5% to 20% of binder, 1% to 2.5% of glidant, 0.2% to 1.2% of lubricant and 0 to 10% of disintegrant. The pharmaceutical composition has excellent stability and a rapid dissolution rate, and is suitable for preparing solid preparations such as quick-release capsules, granules, and tablets. Also provided is a method for preparing a capsule preparation by using the pharmaceutical composition.

## Description

The present application claims priority to the prior application with the patent application No. 2023112633786 and entitled "PHARMACEUTICAL COMPOSITION COMPRISING ALLOPREGNANOLONE DERIVATIVE AND USE THEREOF" filed with the China National Intellectual Property Administration on September 27, 2023, which is incorporated herein by reference in its entirety.

### TECHNICAL FIELD

The present disclosure belongs to the field of pharmaceutical formulations and particularly relates to a pharmaceutical composition comprising an allopregnanolone derivative and use thereof.

### BACKGROUND

It is reported that about 121 million people worldwide are affected by depression. Postpartum depression (PPD) refers to a significant depressive mood disorder that occurs in women during the puerperium, with a global morbidity of about 13% and a morbidity of up to 18.7% in developing countries. PPD usually starts within 4 weeks of giving birth, and the main symptoms include: anxiety, panic, impatience, guilt, sadness, insomnia, fatigue, indifference to the baby, family members, and friends, fear of being alone with the baby, and even suicidal thoughts, which cause great trouble and suffering to the woman who gave birth and her family members.

Allopregnanolone (also known as Brexanolone, tetrahydroprogesterone) is a natural positive allosteric modulator of the human endogenous γ-aminobutyric acid type A receptor (GABAA). In March 2019, the U.S. FDA approved the marketing of a brexanolone intravenous injectable liquid developed by SAGE under the trade name Zulresso^{®} for the treatment of adult PPD. Brexanolone has poor solubility and low oral bioavailability and metabolizes rapidly in the body. Consequently, it is administered by continuous intravenous drip infusion over 60 hours, which greatly inconveniences patients and healthcare professionals.

The applicant's prior patent application WO2023040851A discloses a water-soluble allopregnanolone derivative (compound I) or a pharmaceutically acceptable salt thereof and solvates thereof, and discloses a variety of representative compounds. CN202310230221.7, another prior application, discloses various crystal forms of L-valine-3-deutero, (3α,5α)-3-hydroxy-pregnan-20-one ester hydrochloride (compound II), one of the representative compounds. These compounds have the characteristics of high oral bioavailability, fast onset of action, long acting time, low toxicity and mild side effects, and good metabolic stability, and are suitable for being prepared as oral formulations to improve drug safety, patient compliance, and administration convenience. The two applications described above are incorporated herein by reference in their entirety.

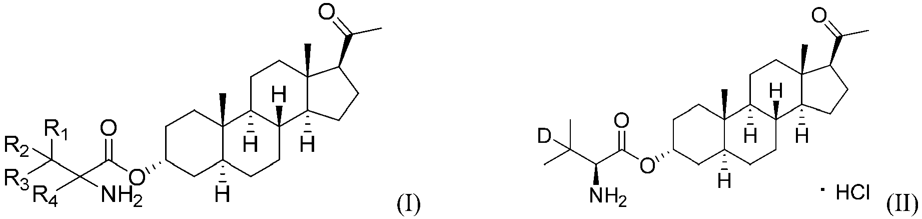

### SUMMARY

The object of the present disclosure is to provide a pharmaceutical composition comprising a water-soluble allopregnanolone derivative, particularly a pharmaceutical composition comprising L-valine-3-deutero, (3α,5α)-3-hydroxy-pregnan-20-one ester hydrochloride (compound II). The pharmaceutical composition has excellent dissolution performance and stability, and is suitable for being prepared as solid formulations such as capsules, granules, and tablets.

The present disclosure provides the following technical solutions:

A pharmaceutical composition, comprising the following components in percentage by weight:

| Component | Percentage by weight |
|---|---|
| Allopregnanolone derivative | 30~70% |
| Filler | 15~62% |
| Binder | 5~20% |
| Glidant | 1~2.5% |
| Lubricant | 0.2~1.2% |
| Disintegrant | 0~10%; |

wherein the allopregnanolone derivative is selected from the following compound (I), a racemate thereof, a stereoisomer thereof, a tautomer thereof, a solvate thereof, a polymorph thereof, or a pharmaceutically acceptable salt thereof: 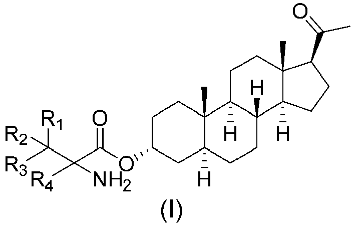
in compound (I), R₂ and R₄ are each independently selected from H (hydrogen) or D (deuterium); R₁ and R₃ are each independently selected from CH₃, CH₂D, CHD₂, or CD₃; provided that compound (I) comprises at least one deuterium atom;
preferably, compound (I) is selected from the following compounds 1 to 5, and racemates, stereoisomers, tautomers, solvates, polymorphs, or pharmaceutically acceptable salts of compounds 1 to 5: 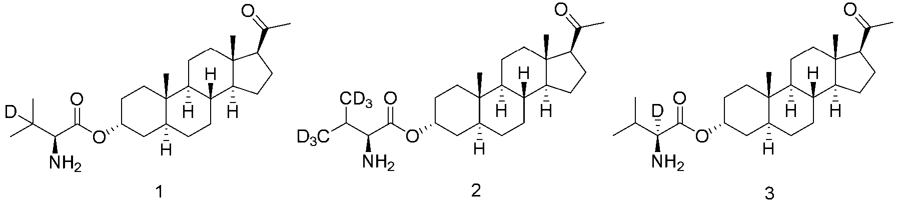 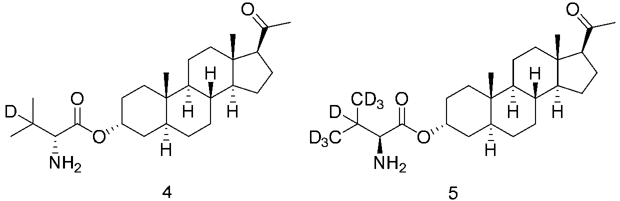
the filler is selected from at least one of microcrystalline cellulose, mannitol, anhydrous dibasic calcium phosphate, sucrose, glucose, or starch;
the binder is selected from at least one of methylcellulose, ethylcellulose, hydroxyethylcellulose, propylcellulose, hydroxypropylcellulose, hydroxypropyl methylcellulose, or pregelatinized starch;
the glidant is selected from at least one of colloidal silica, magnesium stearate, calcium stearate, stearic acid, talc, magnesium carbonate, calcium silicate, or polyethylene glycol;
the lubricant is selected from at least one of stearic acid, magnesium stearate, calcium stearate, aluminum stearate, or talc;
the disintegrant is selected from at least one of crospovidone, dry starch, sodium carboxymethyl starch, low-substituted hydroxypropylcellulose, or croscarmellose sodium.

According to an embodiment of the present disclosure, the filler is selected from a composition of mannitol with one or more of the following substances: microcrystalline cellulose, anhydrous dibasic calcium phosphate, sucrose, glucose, or starch.

In some embodiments of the present disclosure, when the pharmaceutical composition comprises mannitol, the pharmaceutical composition does not comprise a disintegrant.

According to an embodiment of the present disclosure, the content of the filler mannitol is not more than 30 wt%, e.g., 5-30 wt%.

In one embodiment of the present disclosure, the filler is selected from a combination of mannitol and microcrystalline cellulose.

According to an embodiment of the present disclosure, when the composition comprises magnesium stearate, the content of magnesium stearate is 0.2-1.2 wt%, preferably 0.2-1 wt%, e.g., 0.2-0.8 wt%. According to an embodiment of the present disclosure, the allopregnanolone derivative represented by formula (I) is a compound disclosed in WO2023040851A and CN202211112060.3. Accordingly, both patents are incorporated herein by reference in their entirety.

According to an embodiment of the present disclosure, the pharmaceutically acceptable salt is selected from hydrochloride, phosphate, benzoate, *p*-toluenesulfonate, malate, acetate, benzenesulfonate, or maleate.

According to an embodiment of the present disclosure, the allopregnanolone derivative may be the following compound (II):

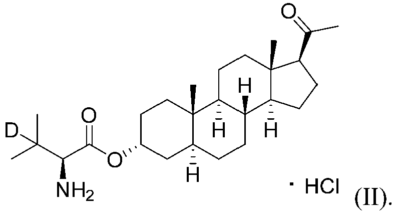

In some embodiments, the pharmaceutical composition comprises the following components in percentage by weight:

| Component | Percentage by weight |
|---|---|
| Compound (II) | 30~70% |
| Microcrystalline cellulose | 10~32% |
| Mannitol | 5~30% |
| Ethylcellulose | 5~20% |
| Colloidal silica | 1~2.5% |
| Magnesium stearate | 0.2~1%. |

In some embodiments, the pharmaceutical composition comprises the following components in percentage by weight:

| Component | Percentage by weight |
|---|---|
| Compound (II) | 50~70% |
| Microcrystalline cellulose | 10~20% |
| Mannitol | 5~15% |
| Ethylcellulose | 10~20% |
| Colloidal silica | 1~2.5% |
| Magnesium stearate | 0.2~1%. |

In some preferred embodiments, the pharmaceutical composition comprises the following components in percentage by weight:

| Component | Percentage by weight |
|---|---|
| Compound (II) | 60~70% |
| Microcrystalline cellulose | 10~15% |
| Mannitol | 5~10% |
| Ethylcellulose | 10~15% |
| Colloidal silica | 1.5~2.5% |
| Magnesium stearate | 0.3~0.8%. |

In some embodiments, the pharmaceutical composition comprises the following components in percentage by weight:

| Component | Percentage by weight |
|---|---|
| Compound (II) | 25~35% |
| Microcrystalline cellulose | 25~35% |
| Mannitol | 15~30% |
| Ethylcellulose | 5~10% |
| Colloidal silica | 1~2.5% |
| Magnesium stearate | 0.2~1%. |

In some preferred embodiments, the pharmaceutical composition comprises the following components in percentage by weight:

| Component | Percentage by weight |
|---|---|
| Compound (II) | 30~35% |
| Microcrystalline cellulose | 25~35% |
| Mannitol | 25~30% |
| Ethylcellulose | 5~8% |
| Colloidal silica | 1~1.5% |
| Magnesium stearate | 0.3~0.8%. |

In some preferred embodiments, the pharmaceutical composition comprises the following components in percentage by weight:

| Component | Percentage by weight |
|---|---|
| Compound (II) | 50~70% |
| Microcrystalline cellulose | 10~20% |
| Mannitol | 5~10% |
| Ethylcellulose | 5~15% |
| Crospovidone | 5~10% |
| Colloidal silica | 1~2.5% |
| Magnesium stearate | 0.2~1%. |

In some preferred embodiments, the pharmaceutical composition comprises the following components in percentage by weight:

| Component | Percentage by weight |
|---|---|
| Compound (II) | 30~35% |
| Microcrystalline cellulose | 25~35% |
| Mannitol | 10~20% |
| Anhydrous dibasic calcium phosphate | 5~15% |
| Ethylcellulose | 5~8% |
| Colloidal silica | 1~1.5% |
| Magnesium stearate | 0.3~0.8%. |

According to an embodiment of the present disclosure, the allopregnanolone derivative may be a crystal form I of compound (II). Patent document CN202310230221.7 discloses a preparation method for the crystal form I of compound (II), and the application is incorporated herein by reference in its entirety.

According to an embodiment of the present disclosure, the crystal form I of compound (II) has an X-ray powder diffraction pattern comprising absorption peaks at the following 2θ angles:

| No. | 2θ±0.2° | d value | Relative intensity | No. | 2θ±0.2° | d value | Relative intensity |
|---|---|---|---|---|---|---|---|
| 1 | 6.27 | 14.08 | 14.1% | 7 | 15.12 | 5.85 | 74.3% |
| 2 | 10.90 | 8.11 | 31.8% | 8 | 15.77 | 5.61 | 36.5% |
| 3 | 12.02 | 7.35 | 4.90% | 9 | 17.35 | 5.11 | 24.8% |
| 4 | 13.27 | 6.67 | 54.0% | 10 | 17.78 | 4.98 | 100.0% |
| 5 | 13.49 | 6.56 | 62.2% | 11 | 20.86 | 4.26 | 14.0% |
| 6 | 14.24 | 6.22 | 42.7% | 12 | 23.95 | 3.71 | 16.2% |

Preferably, the crystal form I has an XRPD pattern substantially as shown in FIG. 1, or further has one or more of the following features: 1) the crystal form I has a DSC thermogram comprising an endothermic peak in a range of about 156±2 °C; 2) the crystal form I has a DSC thermogram substantially as shown in FIG. 2; 3) the crystal form I has a TGA profile substantially as shown in FIG. 3; and 4) the crystal form I has a DVS profile substantially as shown in FIG. 4.

The pharmaceutical composition of the present disclosure may further comprise other excipients, such as a disintegrant and a flavoring agent. However, the inventors have found that when mannitol is used as a filler, an excellent dissolution can be achieved without the addition of a disintegrant. Moreover, in the case of the same content, when a disintegrant is used and mannitol is not added, the dissolution performance is relatively poor.

The pharmaceutical composition of the present disclosure may be formulated as an immediate-release solid formulation, such as an immediate-release capsule, granule, tablet, or the like, preferably a granule or a capsule containing the granule. The capsule may have a capsule shell known in the art, including soft capsule shells and hard capsule shells. For example, the capsule shell is a hard capsule shell, such as a gelatin capsule shell or a plant-based hard capsule shell.

Based on the difference in the content of the active ingredient in a unit formulation, the pharmaceutical composition of the present disclosure may be prepared in different pharmaceutical strengths; for example, the unit formulation comprises 25 mg, 50 mg, 200 mg, or the like, of the active ingredient.

Thus, the present disclosure provides a capsule formulation comprising the pharmaceutical composition according to any one of the above.

Further, the present disclosure provides a method for preparing the capsule formulation described above, comprising the following steps:
1) sieving: sieving the active ingredient compound (I) and excipients separately to obtain sieved materials;
2) premixing: thoroughly mixing the sieved compound (I), other fillers other than mannitol, the glidant, the binder, and optionally part of mannitol to obtain a premixed material;
3) granulation: granulating the premixed material to obtain a granulated material;
4) final mixing: thoroughly mixing the granulated material with the lubricant and all or part of mannitol to obtain a final mixture; and
5) capsule filling: filling capsule shells with the final mixture;
wherein in step 3), the granulation is dry granulation.

According to an embodiment of the present disclosure, when the content of mannitol is less than 15 wt% of the pharmaceutical composition, mannitol may be added in step 4) final mixing; when the content of mannitol is not less than 15 wt%, mannitol is separately added in steps 2) and 4); that is, in step 4), the amount of mannitol added accounts for less than 15 wt%, preferably 5-10 wt%, of the pharmaceutical composition.

In step 4), the amount of mannitol added accounts for less than 15%, preferably 5-10%, by weight of the pharmaceutical composition. According to different pharmaceutical strengths, the content of the filler mannitol may be adjusted as the content of the active ingredient compound changes, while the excellent dissolution and stability of the pharmaceutical composition or formulation are maintained. For example, in a formulation in a relatively large strength (for example, a unit formulation comprises 50 mg or more of the active ingredient), the content of mannitol may account for 5-15% by weight of the pharmaceutical composition; in a formulation in a relatively small strength (for example, a unit formulation comprises 25 mg of the active ingredient), the content of mannitol may account for 20-30% by weight of the pharmaceutical composition.

However, the inventors have also found that during the preparation of solid (capsule) formulations, the amount of mannitol added affects the performance of formulation granules.

When all mannitol is added in step 2) premixing, the dissolution of the active ingredient in the resulting formulation is significantly relatively slow; preferably, when the content of mannitol in the pharmaceutical composition is less than 15 wt%, adding mannitol in step 4) final mixing when a capsule is prepared can significantly increase the dissolution of the drug.

When the content of mannitol in the pharmaceutical composition is relatively high (e.g., up to 15 wt%, 20 wt%, 25 wt%, or higher), if all mannitol is added in step 4) final mixing, the content uniformity of the resulting formulation easily exceeds the acceptance criteria in the test for content uniformity. The inventors have found that when the mannitol content is relatively high, mannitol is preferably added in two portions, of which one is added in the premixing step, and the other is added in the final mixing step. In some specific embodiments of the present disclosure, the content of mannitol added in the final mixing step is less than 15%, preferably no more than 10%, of the total weight of the pharmaceutical composition, and the remaining mannitol is added in the premixing step, which can solve the problem described above, significantly improve the uniformity of drug granules, and improve the smoothness and controllability of the formulation process.

The present disclosure further provides use of the pharmaceutical composition or the solid formulation (e.g., capsule, granule, tablet, or the like) for the manufacturing of a medicament for the prevention or treatment of central nervous system-related diseases, or for the manufacturing of a medicament for sedation and hypnosis.

According to an embodiment of the present disclosure, the central nervous system-related diseases are, for example, traumatic brain injury, essential tremor, epilepsy (including refractory persistent epilepsy), rare genetic epilepsy (e.g., Dravet syndrome and Rett syndrome), depression (including postpartum depression), and Alzheimer's disease. For example, the central nervous system diseases are selected from essential tremor, epilepsy, clinical depression, postnatal or postpartum depression, atypical depression, psychotic major depression, catatonic depression, seasonal affective disorder, dysthymia, double depression, depressive personality disorder, recurrent transient depression, minor depressive disorder, bipolar disorder or manic depressive disorder, post-traumatic stress disorder, depression caused by chronic medical conditions, treatment-resistant depression, refractory depression, suicidal tendency, suicidal ideations, and suicidal behaviors.

In the pharmaceutical composition of the present disclosure, compound (I) has excellent stability and dissolution performance as an active ingredient. After oral administration, compound (I) is hydrolyzed and rapidly releases the active metabolite allopregnanolone. Oral administration of allopregnanolone is achieved by means of a prodrug. Compared to Zulresso^{®}, the solid formulation of compound (I) can improve the convenience of clinical use, improve patient compliance, reduce the risk of administration, reduce the length of hospital stay, and be more in line with clinical practice, while maintaining efficacy.

### BRIEF DESCRIPTION OF THE DRAWINGS

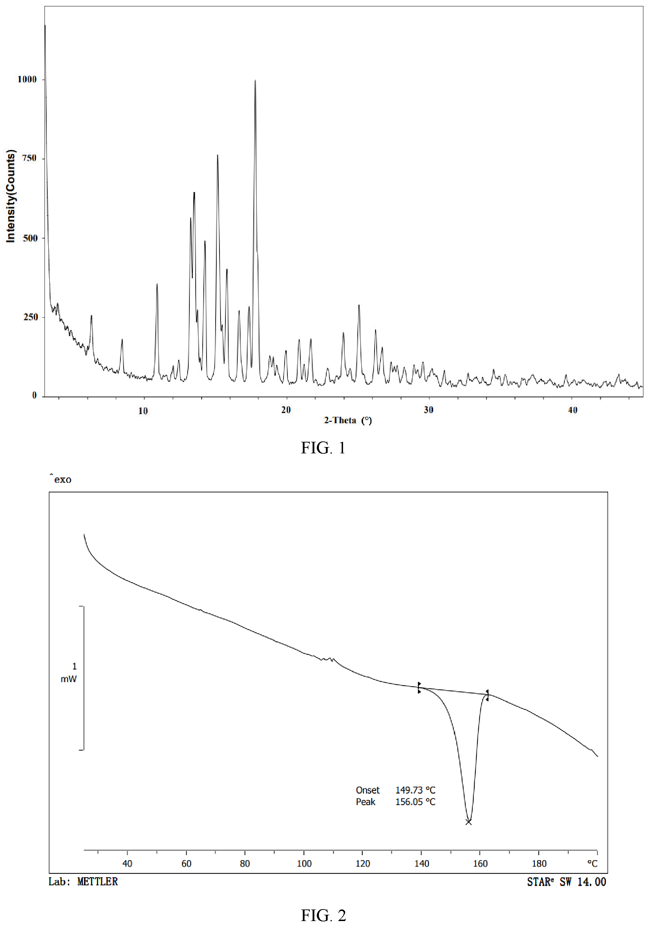

FIG. 1 shows an XRPD pattern of the crystal form I of compound (II).
FIG. 2 shows a DSC thermogram of the crystal form I of compound (II).

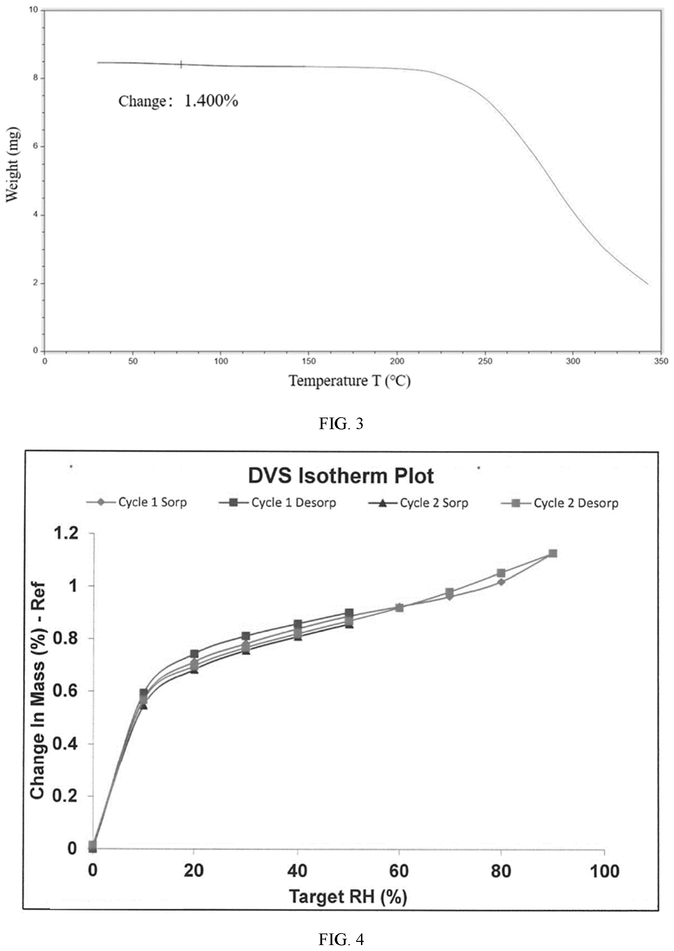

FIG. 3 shows a TGA profile of the crystal form I of compound (II).
FIG. 4 shows a DVS profile of the crystal form I of compound (II).

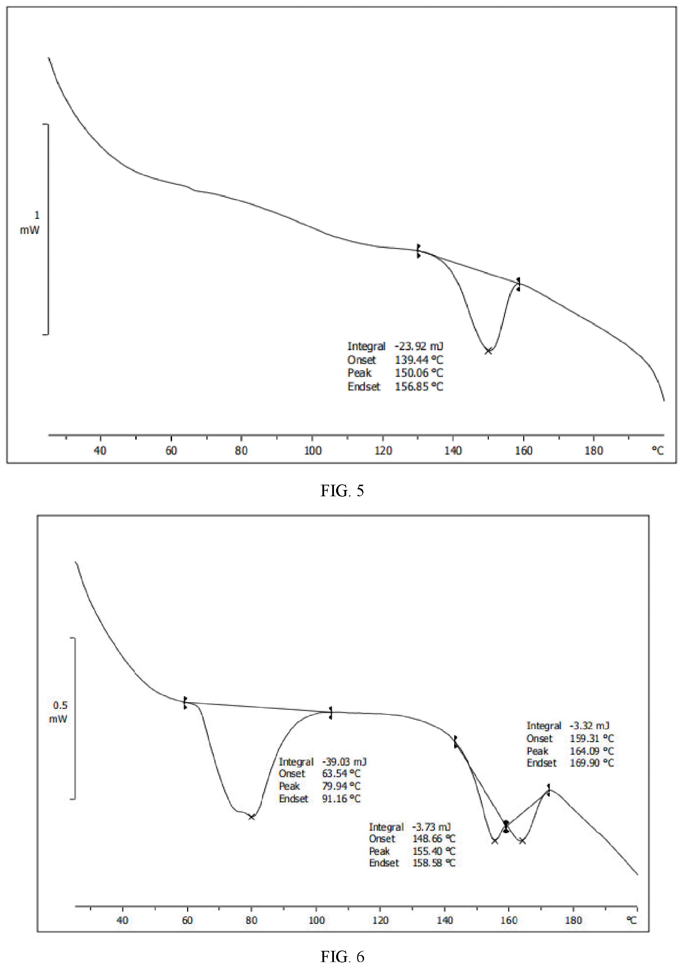

FIG. 5 is a DSC thermogram of the granules of Example 10 (dry granulation).
FIG. 6 is a DSC thermogram of the granules of Comparative Example 8 (wet granulation).

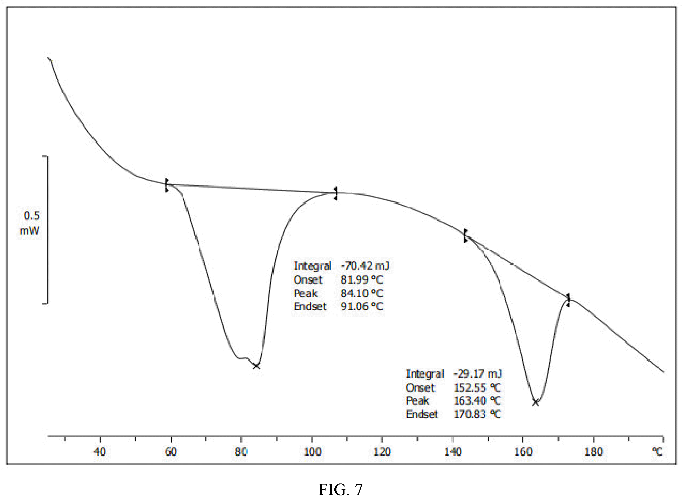

FIG. 7 is a DSC thermogram of the granules of Comparative Example 9 (wet granulation).

### DETAILED DESCRIPTION

The technical solutions of the present disclosure are described below in as much detail as possible through examples, with compound (II) as a representative compound. The examples should not be construed as any limitation on the present disclosure. Unless otherwise specified, the materials used in the examples were all commercially available, and the experimental procedures used were all performed according to methods or instruments known or conventional in the art.

Compound (II) sample: The crystal form I of compound (II) can be prepared by referring to the method described in patent document CN202310230221.7. About 5.0 g of compound (II) was added to 50 mL of isopropyl acetate. The mixture was stirred for trituration at 50 °C and filtered. The filter cake was washed with 10 mL of isopropyl acetate and dried *in vacuo* at 50 °C to give the crystal form I of compound (II). The crystal form I has an XRPD pattern substantially as shown in FIG. 1; its DSC thermogram has an endothermic peak in a range of about 156±2 °C; its DSC thermogram is specifically shown in FIG. 2. Its TGA profile is shown in FIG. 3. The crystal form I further has a DVS profile substantially as shown in FIG. 4.

Preparation of capsules: In the following examples, capsules were prepared according to the formulations shown in the tables, with a batch size of 1200 capsules/batch.

Stability test: 1) Samples were tested by storage at 40 °C in a 75% RH environment; 2) samples were tested by storage at 60 °C in a dry environment. HPLC detection conditions for related substances: mobile phase A: 10 mmol/L diammonium hydrogen phosphate solution, mobile phase B: acetonitrile; diluent: 70% methanol solution; detection wavelength: 205 nm.

Dissolution test: 0.01 N hydrochloric acid medium; paddle method (sinker); rotation speed: 50 rpm; volume: 900 mL; temperature: 37±0.5 °C; sampling time points: 10 min, 15 min, 20 min, 30 min, and 45 min.

Mixing uniformity test: At the end of mixing, samples are taken from a total of 11 positions, including the upper part of the mixing bucket, corner edges, and the discharge port, and the content in the samples was determined; the individual content values in the mixing uniformity samples should be within ±10% (absolute) of the mean, and the RSD value should be ≤5.0%.

Content uniformity test: 10 formulation samples were taken, and the contents of each sample were accurately transferred to a measuring flask. A proper amount of a diluent was added, and the mixture was ultrasonicated while being shaken to dissolve the contents, left to cool, and diluted to volume with the diluent. The dilution was made homogeneous by shaking and filtered through a filter membrane, and the filtrate was collected. The content uniformity of the product was tested according to General Chapter <0941> in Volume IV of the Chinese Pharmacopoeia, 2020 Edition. The mean content and standard deviation S, as well as the absolute value A of the difference between the labeled amount and the mean value, were calculated, and the results were judged according to the acceptance criteria. The criteria were as follows: if A + 2.2S ≤ L, the content uniformity of the test sample meets the requirements; if A + S > L, the test sample does not meet the requirements; L = 15.0.

DSC testing parameters: Samples were placed in an aluminum oxide crucible for DSC testing, and the parameters were set as follows: heating range: 25-200 °C; heating rate: 10 °C/min; nitrogen purge gas: 50 mL/min.

### Examples 1-2 and Comparative Example 1: Preparation of Capsule Formulations with Pharmaceutical Composition of Present Disclosure

Example 1, Example 2, and Comparative Example 1 show the effects of different contents of the components, particularly mannitol, on the pharmaceutical composition of the present disclosure. The formulations are shown in Table 2 below.

Preparation method: 1) sieving: the amounts of compound (II) and the excipients were separately sieved (40-mesh standard sieve; the same applies hereinafter) to obtain sieved materials; 2) premixing: the sieved microcrystalline cellulose, colloidal silica, compound (II), and ethylcellulose were thoroughly mixed to obtain a premixed material; 3) granulation: the premixed material was rolled using a dry granulator to obtain a granulated material; 4) final mixing: the granulated material was thoroughly mixed with additional excipients (magnesium stearate and mannitol) to obtain a final mixture; and 5) capsule filling: gelatin capsule shells were filled with the final mixture.

**Table 2**

| Function | Component | Example 1 | Example 2 | Comparative Example 1 |
|---|---|---|---|---|
| Active ingredient | Compound (II) | 60.0 | 70.0 | 70.0 |
| Filler | Microcrystalline cellulose | 15.0 | 10.0 | 15.0 |
| Binder | Ethylcellulose | 15.0 | 10.0 | 12.0 |
| Glidant | Colloidal silica | 2.0 | 1.5 | 2.0 |
| Lubricant | Magnesium stearate | 0.5 | 1.0 | 1.0 |
| Filler | Mannitol | 7.5 | 7.5 | 0 |
| | Total (g) | 100 | 100 | 100 |

The dissolution test results are shown in Table 3.

**Table 3**

| Time | 10 min | 15 min | 20 min | 30 min | 45 min |
|---|---|---|---|---|---|
| Example 1 | 52.0% | 71.4% | 85.6% | 97.7% | 97.9% |
| Example 2 | 56.9% | 71.3% | 90.0% | 97.3% | 97.2% |
| Comparative Example 1 | 6.5% | 23.4% | 44.5% | 85.2% | 93.1% |

The stability test results are shown in Table 4 below.

**Table 4**

| | | 0 days | 40 °C/75% RH, 30 days | 60 °C, 30 days |
|---|---|---|---|---|
| Example 1 | Maximum single impurity (%) | 0.11 | 0.10 | 0.11 |
| | Total impurity (%) | 0.19 | 0.18 | 0.22 |
| Example 2 | Maximum single impurity (%) | 0.11 | 0.14 | 0.11 |
| | Total impurity (%) | 0.24 | 0.24 | 0.25 |
| Comparative Example 1 | Maximum single impurity (%) | 0.11 | 0.09 | 0.13 |
| | Total impurity (%) | 0.19 | 0.17 | 0.24 |

The results show that the dissolution of the formulation of Comparative Example 1 was about 85% at 30 min and about 93% at 45 min; in contrast, the dissolution of the formulations of Examples 1 and 2 were more than 80% at 20 min and more than 97% at 30 min, which were significantly higher than those of Comparative Example 1. It can be seen that the addition of mannitol was conducive to the rapid dissolution and release of the API compared to the formulation without mannitol. Moreover, the formulations of Examples 1 and 2 both showed good stability at 40 °C/75% RH and at a high temperature of 60 °C. In addition, the tests showed that the mixing uniformity and content uniformity of the samples of Examples 1 and 2 both met the criteria.

### Examples 3-4 and Comparative Example 2: Preparation of Capsule Formulations with Pharmaceutical Composition of Present Disclosure

Examples 3 and 4 and Comparative Example 2 show the effects of the filler mannitol, the disintegrant crospovidone, etc., on the pharmaceutical composition. The formulations are shown in Table 5. Preparation method: the same as that of Example 1.

**Table 5**

| Function | Component | Example 3 | Example 4 | Comparative Example 2 |
|---|---|---|---|---|
| Active ingredient | Compound (II) | 66.0 | 66.0 | 66.0 |
| Filler | Microcrystalline cellulose | 12.5 | 10.0 | 12.5 |
| Binder | Ethylcellulose | 12.5 | 10.0 | 12.5 |
| Glidant | Colloidal silica | 1.5 | 1.5 | 1.5 |
| Lubricant | Magnesium stearate | 0.5 | 0.5 | 0.5 |
| Filler | Mannitol | 7.0 | 6.0 | 0 |
| Disintegrant | Crospovidone | 0 | 6.0 | 7.0 |
| | Total (g) | 100 | 100 | 100 |

The dissolution test results are shown in Table 6.

**Table 6**

| Time | 10 min | 15 min | 20 min | 30 min | 45 min |
|---|---|---|---|---|---|
| Example 3 | 51.0% | 69.0% | 82.2% | 96.6% | 99.5% |
| Example 4 | 50.6% | 65.0% | 79.9% | 97.0% | 100.3% |
| Comparative Example 2 | 32.3% | 55.9% | 66.6% | 81.4% | 94.2% |

The stability test results are shown in Table 7 below.

**Table 7**

| | | 0 days | 40 °C/75% RH, 30 days | 60 °C, 30 days |
|---|---|---|---|---|
| Example 3 | Maximum single impurity (%) | 0.10 | 0.13 | 0.13 |
| | Total impurity (%) | 0.20 | 0.25 | 0.26 |
| Example 4 | Maximum single impurity (%) | 0.11 | 0.12 | 0.12 |
| | Total impurity (%) | 0.22 | 0.21 | 0.22 |
| Comparative Example 2 | Maximum single impurity (%) | 0.12 | 0.12 | 0.11 |
| | Total impurity (%) | 0.23 | 0.21 | 0.24 |

The experimental results show that the formulations of Examples 3 and 4 both contained mannitol, and their dissolutions were more than 95% at 30 min; Example 4 also contained the disintegrant crospovidone, and its dissolution was not significantly different from that of Example 3. The formulation of Comparative Example 2 did not contain mannitol and only contained crospovidone, and its dissolution at 30 min was significantly lower than those of Examples 3 and 4. The results described above show that mannitol promoted the rapid dissolution of the API in the pharmaceutical composition of the present disclosure, and although the disintegrant crospovidone promoted the dissolution of the API, its effect was significantly inferior to that of mannitol. In the case that mannitol is added, the addition of the disintegrant crospovidone may not be required. In addition, the formulations of Examples 3 and 4 both showed good stability, and the tests showed that their mixing uniformity and content uniformity both met the criteria.

### Examples 5-6 and Comparative Example 3: Pharmaceutical Composition

Examples 5 and 6 and Comparative Example 3 show the effects of different filler contents on the pharmaceutical composition. The formulations are shown in Table 8.

Preparation method: the same as that of Example 1.

**Table 8**

| Function | Component | Example 5 | Example 6 | Comparative Example 3 |
|---|---|---|---|---|
| Active ingredient | Compound (II) | 30.0 | 35.0 | 30.0 |
| Filler | Microcrystalline cellulose | 30.5 | 32.5 | 0 |
| Filler | Silicified microcrystalline cellulose | 0 | 0 | 30.0 |
| Filler | Lactose | 0 | 0 | 28.3 |
| Binder | Ethylcellulose | 8.0 | 6.0 | 10.0 |
| Filler | Mannitol | 30.0 | 15.0 | 0 |
| Filler | Anhydrous dibasic calcium phosphate | 0 | 10.0 | 0 |
| Glidant | Colloidal silica | 1.2 | 1.0 | 1.2 |
| Lubricant | Magnesium stearate | 0.3 | 0.5 | 0.5 |
| | Total (g) | 100 | 100 | 100 |

The dissolution test results are shown in Table 9.

**Table 9**

| Time | 10 min | 15 min | 20 min | 30 min | 45 min |
|---|---|---|---|---|---|
| Example 5 | 75.2% | 80.6% | 88.5% | 98.6% | 99.1% |
| Example 6 | 68.0% | 79.0% | 89.2% | 96.6% | 99.5% |
| Comparative Example 3 | 65.8% | 77.5% | 88.3% | 96.2% | 98.2% |

The stability test results are shown in Table 10 below.

**Table 10**

| | | 0 days | 40 °C/75% RH, 30 days | 60 °C, 30 days |
|---|---|---|---|---|
| Example 5 | Maximum single impurity (%) | 0.11 | 0.11 | 0.11 |
| | Total impurity (%) | 0.19 | 0.20 | 0.21 |
| Example 6 | Maximum single impurity (%) | 0.12 | 0.24 | 0.26 |
| | Total impurity (%) | 0.18 | 0.51 | 0.43 |
| Comparative Example 3 | Maximum single impurity (%) | 0.10 | 0.26 | 0.81 |
| | Total impurity (%) | 0.20 | 0.57 | 1.07 |

The experimental results show that the formulation of Example 5 contained microcrystalline cellulose and mannitol as fillers, the formulation of Example 6 contained microcrystalline cellulose, mannitol, and anhydrous dibasic calcium phosphate as fillers, and the formulation of Comparative Example 3 contained silicified microcrystalline cellulose and lactose as fillers; their dissolutions were about 96%-98% at 30 min. Comparison shows that there is no significant difference in the dissolution of the three. However, Comparative Example 3 showed poor stability at 40 °C/75% RH and at a high temperature of 60 °C, and the stability of Examples 5 and 6 was significantly superior to that of Comparative Example 3. Therefore, silicified microcrystalline cellulose and lactose are not suitable as fillers in the pharmaceutical composition of the present disclosure. The filler of the pharmaceutical composition of the present disclosure may be selected from microcrystalline cellulose, mannitol, and anhydrous dibasic calcium phosphate, more preferably from microcrystalline cellulose and mannitol. In addition, the tests showed that the mixing uniformity and content uniformity of the formulations of Examples 5 and 6 both met the criteria.

### Examples 7-8 and Comparative Example 4

Examples 7 and 8 and Comparative Example 4 show the effects of different preparation methods (especially the way of adding mannitol) on the preparation of the pharmaceutical composition as capsule granules. The formulations are shown in Table 11 below.

Preparation method of Example 7: the same as that of Example 1. Mannitol was added in step 4) final mixing.

Preparation method of Example 8: substantially the same as that of Example 7, with the difference being that the amount of mannitol was equally divided into two portions, of which one was added in step 2) premixing, and the other was added in step 4) final mixing.

Preparation method of Comparative Example 4: substantially the same as that of Example 7, with the difference being that the amount of mannitol was all added in step 2) premixing.

**Table 11**

| Component | Amount |
|---|---|
| Compound (II) | 66.0 |
| Microcrystalline cellulose | 12.0 |
| Ethylcellulose | 12.0 |
| Colloidal silica | 2.0 |
| Magnesium stearate | 0.6 |
| Mannitol | 7.4 |
| Total (g) | 100 |

The dissolution test results are shown in Table 12.

**Table 12**

| Time | 10 min | 15 min | 20 min | 30 min | 45 min |
|---|---|---|---|---|---|
| Example 7 | 51.9% | 72.0% | 86.2% | 97.6% | 99.5% |
| Example 8 | 40.3% | 46.5% | 63.3% | 90.6% | 97.1% |
| Comparative Example 4 | 31.4% | 39.8% | 60.8% | 86.7% | 97.3% |

The experimental results show that the dissolution of Example 7, in which mannitol was added in the final mixing step, was 97.6% at 30 min and was the most rapid. In Example 8, part of mannitol (3.7 wt%) was added in the premixing step, and the remaining portion (3.7 wt%) was added in the final mixing step; its dissolution in the first 20 min was significantly slower than that of Example 7, and its dissolution was 90.6% at 30 min, which was lower than that of Example 7. In Comparative Example 4, mannitol was all added in the premixing step, and its dissolution was the slowest, indicating that the way of adding mannitol had a certain effect on the dissolution. Mannitol is preferably added in the final mixing step. The tests showed that the mixing uniformity and content uniformity of the formulations both met the criteria.

### Example 9 and Comparative Examples 5 and 6

Example 9 and Comparative Examples 5, 6, and 7 show the effects of different preparation methods on the preparation of the pharmaceutical composition as capsule granules. The formulations are shown in Table 13 below.

Preparation method of Example 9: 1) sieving: the amounts of compound (II) and the other excipients were separately sieved to obtain sieved materials; 2) premixing: the sieved microcrystalline cellulose, part of mannitol (accounting for 19.7 wt% of the weight of the pharmaceutical composition), colloidal silica, compound (II), and ethylcellulose were thoroughly mixed to obtain a premixed material; 3) dry granulation: the premixed material was rolled using a dry granulator to obtain a granulated material; 4) final mixing: the granulated material was mixed with additional excipients [the remaining mannitol (accounting for 10 wt% of the weight of the pharmaceutical composition) and magnesium stearate] to obtain a final mixture; and 5) capsule filling: gelatin capsule shells were filled with the final mixture. Preparation method of Comparative Example 5: substantially the same as that of Example 9, with the difference being that part of mannitol (accounting for 9.7 wt% of the weight of the pharmaceutical composition) was added in step 2) premixing, and the remaining mannitol (accounting for 20 wt% of the weight of the pharmaceutical composition) was added in step 4) final mixing.

Preparation method of Comparative Example 6: substantially the same as that of Example 9, with the difference being that mannitol was all added in step 4) final mixing.

Preparation method of Comparative Example 7: substantially the same as that of Example 9, with the difference being that mannitol was all added in step 2) premixing.

**Table 13**

| Component | Example 9 | Comparative Example 5 | Comparative Example 6 | Comparative Example 7 |
|---|---|---|---|---|
| Compound (II) | 33.0 | 33.0 | 33.0 | 33.0 |
| Microcrystalline cellulose | 30.0 | 30.0 | 15.0 | 15.0 |
| Ethylcellulose | 6.0 | 6.0 | 6.0 | 6.0 |
| Colloidal silica | 1.0 | 1.0 | 1.9 | 1.0 |
| Mannitol | 29.7 | 29.7 | 43.5 | 43.5 |
| Magnesium stearate | 0.3 | 0.3 | 0.6 | 1.5 |
| Total (g) | 100 | 100 | 100 | 100 |

The dissolution test results are shown in Table 14.

**Table 14**

| Time | 10 min | 15 min | 20 min | 30 min | 45 min |
|---|---|---|---|---|---|
| Example 9 | 70.4% | 89.0% | 95.2% | 96.6% | 99.5% |
| Comparative Example 5 | 70.8% | 90.0% | 96.1% | 97.9% | 99.7% |
| Comparative Example 6 | 71.9% | 90.7% | 96.4% | 98.2% | 99.0% |
| Comparative Example 7 | 69.5% | 90.0% | 88.7% | 95.6% | 98.4% |

The mixing uniformity and content uniformity test results are shown in Table 15 below.

**Table 15**

| | Mixing uniformity of final mixed material | Content uniformity of formulation |
|---|---|---|
| Example 9 | Individual content values did not exceed ±10%; the RSD value was ≤5.0%; Met the criteria | A + 2.2S ≤ L; Met the criteria |
| Comparative Example 5 | Individual content values exceeded ± 10%, and the RSD value was >5.0%; Did not meet the criteria | A + S > L; Did not meet the criteria |
| Comparative Example 6 | Individual content values exceeded ± 10%, and the RSD value was >5.0%; Did not meet the criteria | A + S > L; Did not meet the criteria |
| Comparative Example 7 | Individual content values did not exceed ±10%; the RSD value was ≤5.0%; Met the criteria | A + 2.2S ≤ L; Met the criteria |

The stability test results are shown in Table 16 below.

**Table 16**

| | | 0 days | 40 °C/75% RH, 30 days | 60 °C, 30 days |
|---|---|---|---|---|
| Example 9 | Maximum single impurity (%) | 0.11 | 0.12 | 0.12 |
| | Total impurity (%) | 0.20 | 0.20 | 0.23 |
| Comparative Example 5 | Maximum single impurity (%) | 0.10 | 0.11 | 0.13 |
| | Total impurity (%) | 0.20 | 0.21 | 0.22 |
| Comparative Example 6 | Maximum single impurity (%) | 0.10 | 0.12 | 0.11 |
| | Total impurity (%) | 0.20 | 0.22 | 0.22 |
| Comparative Example 7 | Maximum single impurity (%) | 0.12 | 0.12 | 0.34 |
| | Total impurity (%) | 0.22 | 0.24 | 0.56 |

The results show that in Example 9 and Comparative Examples 5-7, the API content was relatively small, the mannitol content was relatively high, and their API dissolutions were all relatively rapid, showing no significant difference. However, the way of adding mannitol affected the mixing uniformity and content uniformity of the composition. When mannitol accounted for 29.7 wt% of the weight of the pharmaceutical composition, if the amount added in the final mixing step was 10 wt%, good uniformity could be obtained during rolling granulation, and the material did not adhere to the roller, resulting in a smooth process (Example 9). However, when the amount of mannitol added in the final mixing step was 20 wt%, the mixing uniformity of the final mixed material and the content uniformity of the formulation did not meet the acceptance criteria in the test for content uniformity in General Chapter <0941> in Volume IV of the Chinese Pharmacopoeia, 2020 Edition (Comparative Example 5).

Further, when the content of mannitol in the pharmaceutical composition reached 43.5 wt%, and mannitol was all added in the final mixing step, the mixing uniformity of the final mixed material and the content uniformity of the formulation did not meet the acceptance criteria in the test for content uniformity in General Chapter <0941> in Volume IV of the Chinese Pharmacopoeia, 2020 Edition (Comparative Example 6); when mannitol was all added in the premixing step, the mixture easily adhered to the roller during rolling granulation, resulting in too much fine powder in the granules and poor granulation uniformity, which affected the smoothness of the process and the uniformity of capsule filling (Comparative Example 7).

Thus, when the mannitol content of the composition is relatively high, mannitol is preferably added in two portions, of which one is added in the premixing step, and the other is added in the final mixing step, wherein the content of mannitol added in the final mixing step is less than 15% of the total weight of the pharmaceutical composition, preferably no more than 10% of the total amount of the pharmaceutical composition, and the remaining mannitol is added in the premixing step, which can significantly improve the uniformity of drug granules and improve the smoothness and controllability of the formulation process.

In addition, the stability test results show that Comparative Example 7 exhibited relatively poor stability at the high temperature. According to further analysis, this was due to the relatively high content of magnesium stearate. It was found through magnesium stearate content screening that in formulations using the same ingredients, when the content of magnesium stearate was more than 1.2 wt% of the total weight of the formulation, the stability of the pharmaceutical composition decreased. Preferably, the content of magnesium stearate in the present disclosure should be no more than 1.2 wt%, more preferably no more than 1 wt%, e.g., 0.2-0.8 wt%.

### Example 10 and Comparative Examples 8 and 9

In Example 10 and Comparative Examples 8 and 9, capsule granules were prepared using the formulations shown in Table 17 below and different granulation methods, and the effects of different preparation processes on the pharmaceutical composition were investigated.

Preparation method of Example 10 (dry granulation): the same as that of Example 1.

Preparation method of Comparative Example 8 (wet granulation): 1) sieving: the amounts of compound (II) and the excipients were separately sieved to obtain sieved materials; 2) premixing: the sieved microcrystalline cellulose, mannitol, colloidal silica, compound (II), and ethylcellulose were mixed to obtain a premixed material; 3) wet granulation: a soft material was prepared using a wet granulator with a proper amount of water as a wetting agent and granulated using a swing granulator, and the material was dried in a drying oven after granulation was completed; 4) sizing and final mixing: the dried material was sized using a sizing machine, and the sized material was mixed with an additional pharmaceutically acceptable excipient (magnesium stearate) to obtain a final mixture; and 5) capsule filling: gelatin capsule shells were filled with the final mixture.

Preparation method of Comparative Example 9 (wet granulation): substantially the same as that of Comparative Example 8, with the difference being that a 40% aqueous ethanol solution was used as a wetting agent.

**Table 17**

| Component | Amount |
|---|---|
| Compound (II) | 35.0 |
| Microcrystalline cellulose | 30.0 |
| Ethylcellulose | 8.0 |
| Colloidal silica | 1.2 |
| Magnesium stearate | 0.3 |
| Mannitol | 25.5 |
| Total (g) | 100 |

The raw material compound (II) and the capsule granules after granulation and drying were tested by differential scanning calorimetry (DSC) to investigate whether the active ingredient compound (II) underwent polymorphic transition before and after granulation. The DSC thermogram of the raw material compound (II) is shown in FIG. 2. The DSC thermograms of the capsule granules of Example 10, Comparative Example 8, and Comparative Example 9 are shown in FIG. 5 to FIG. 7, respectively. The experimental results show that the raw material compound (II) was in crystal form I, and its DSC thermogram had an endothermic peak at about 156.05 °C. In Example 10, in which dry granulation was used, the crystal form of the API in the obtained granules had an endothermic peak at 150.06 °C, and there were no other new endothermic peaks, indicating that the crystal form of compound (II) did not transition (the relatively small difference in the endothermic peak compared to the profile of the drug substance was due to the interference of the excipients in the pharmaceutical composition); in Comparative Example 8, in which wet granulation was performed with water as an binder, new crystal form endothermic peaks (about 79.94 °C and 164.09 °C) appeared in the DSC thermogram, indicating that the crystal form I of the API in the granules partially transitioned; in Comparative Example 9, in which wet granulation was performed with a 40% aqueous ethanol solution as an binder, the melting point endothermic peak of the crystal form I disappeared from the DSC thermogram, and only new endothermic peaks (at about 84.10 °C and 163.40 °C) were present, indicating that all of the API in the granules underwent polymorphic transition. Through the above experiments, it can be determined that the wet granulation process can cause the crystal form of the active ingredient to transition, leading to an unstable formulation. Thus, wet granulation is not suitable for the formulation process of this product. Therefore, dry granulation is preferably used in the preparation of the pharmaceutical composition of the present disclosure as a solid formulation.

In summary, the pharmaceutical composition of the present disclosure has excellent dissolution performance and stability, and when the pharmaceutical composition is prepared as solid dosage forms such as capsules by the preparation method of the present disclosure, the active ingredient will not undergo polymorphic transition; moreover, the formulation process is simple and smooth, without sticking to rollers or excessive fine powder, and can yield uniform and stable granules.

The examples described above are used to exemplify the technical solutions of the present disclosure. Those skilled in the art can make modifications based on the present disclosure, and the modified technical solutions shall fall within the protection scope of the present disclosure, provided that they do not depart from the spirit of the present disclosure.

## Claims

1. A pharmaceutical composition, comprising the following components in percentage by weight:
| Component | Percentage by weight |
|---|---|
| Allopregnanolone derivative | 30~70% |
| Filler | 15~62% |
| Binder | 5~20% |
| Glidant | 1~2.5% |
| Lubricant | 0.2~1.2% |
| Disintegrant | 0~10% |
wherein the allopregnanolone derivative is selected from compound (I), a racemate thereof, a stereoisomer thereof, a tautomer thereof, a solvate thereof, a polymorph thereof, or a pharmaceutically acceptable salt thereof: 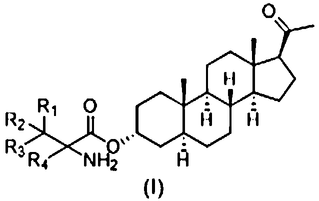 in compound (I), R₂ and R₄ are each independently selected from H (hydrogen) or D (deuterium); R₁ and R₃ are each independently selected from CH₃, CH₂D, CHD₂, or CD₃; provided that compound (I) comprises at least one deuterium atom.

2. The pharmaceutical composition as claimed in claim 1, wherein the allopregnanolone derivative is selected from one or more of the following compounds 1 to 5, and racemates, stereoisomers, tautomers, solvates, polymorphs, or pharmaceutically acceptable salts thereof: 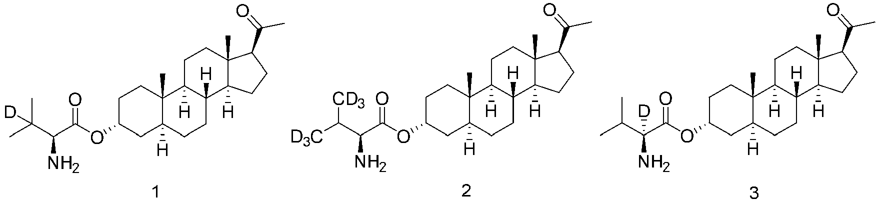 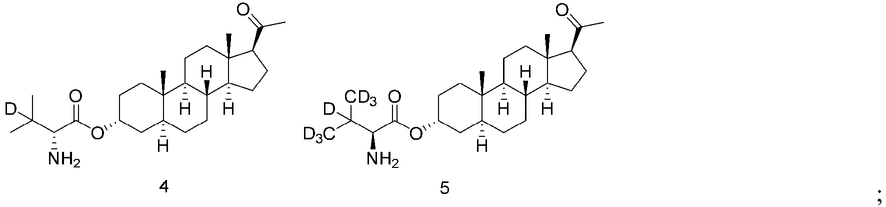 preferably, the allopregnanolone derivative is selected from the following compound (II):

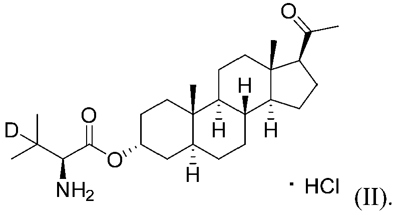

3. The pharmaceutical composition as claimed in claim 1 or 2, wherein the filler is selected from one or more of microcrystalline cellulose, mannitol, sucrose, glucose, and starch; and/or
the binder is selected from one or more of methylcellulose, ethylcellulose, hydroxyethylcellulose, propylcellulose, hydroxypropylcellulose, hydroxypropyl methylcellulose, or pregelatinized starch; and/or
the glidant is selected from one or more of colloidal silica, magnesium stearate, calcium stearate, stearic acid, talc, magnesium carbonate, calcium silicate, or polyethylene glycol; and/or the lubricant is selected from one or more of stearic acid, magnesium stearate, calcium stearate, aluminum stearate, or talc; and/or
the disintegrant is selected from one or more of crospovidone, dry starch, sodium carboxymethyl starch, low-substituted hydroxypropylcellulose, or croscarmellose sodium;
preferably, the filler is selected from a composition of mannitol with one or more of the following substances: microcrystalline cellulose, anhydrous dibasic calcium phosphate, sucrose, glucose, or starch;
preferably, when the pharmaceutical composition comprises mannitol, the pharmaceutical composition does not comprise a disintegrant.

4. The pharmaceutical composition as claimed in any one of claims 1-3, wherein the pharmaceutical composition comprises the following components in percentage by weight:
| Component | Percentage by weight |
|---|---|
| Compound (II) | 30~70% |
| Microcrystalline cellulose | 10~32% |
| Mannitol | 5~30% |
| Ethylcellulose | 5~20% |
| Colloidal silica | 1~2.5% |
| Magnesium stearate | 0.2~1%; |
preferably, the pharmaceutical composition comprises the following components in percentage by weight:
| Component | Percentage by weight |
|---|---|
| Compound (II) | 50~70% |
| Microcrystalline cellulose | 10~20% |
| Mannitol | 5~15% |
| Ethylcellulose | 10~20% |
| Colloidal silica | 1~2.5% |
| Magnesium stearate | 0.2~1%; |
preferably, the pharmaceutical composition comprises the following components in percentage by weight:
| Component | Percentage by weight |
|---|---|
| Compound (II) | 60~70% |
| Microcrystalline cellulose | 10~15% |
| Mannitol | 5~10% |
| Ethylcellulose | 10~15% |
| Colloidal silica | 1.5~2.5% |
| Magnesium stearate | 0.3~0.8%; |
preferably, the pharmaceutical composition comprises the following components in percentage by weight:
| Component | Percentage by weight |
|---|---|
| Compound (II) | 25~35% |
| Microcrystalline cellulose | 25~35% |
| Mannitol | 15~30% |
| Ethylcellulose | 5~10% |
| Colloidal silica | 1~2.5% |
| | |
|---|---|
| Magnesium stearate | 0.2~1%; |
preferably, the pharmaceutical composition comprises the following components in percentage by weight:
| Component | Percentage by weight |
|---|---|
| Compound (II) | 30~35% |
| Microcrystalline cellulose | 25~35% |
| Mannitol | 25~30% |
| Ethylcellulose | 5~8% |
| Colloidal silica | 1~1.5% |
| Magnesium stearate | 0.3~0.8%; |
preferably, the pharmaceutical composition comprises the following components in percentage by weight:
| Component | Percentage by weight |
|---|---|
| Compound (II) | 50~70% |
| Microcrystalline cellulose | 10~20% |
| Mannitol | 5~10% |
| Ethylcellulose | 5~15% |
| Crospovidone | 5~10% |
| Colloidal silica | 1~2.5% |
| Magnesium stearate | 0.2~1%; |
preferably, the pharmaceutical composition comprises the following components in percentage by weight:
| Component | Percentage by weight |
|---|---|
| Compound (II) | 30~35% |
| Microcrystalline cellulose | 25~35% |
| Mannitol | 10~20% |
| Anhydrous dibasic calcium phosphate | 5~15% |
| Ethylcellulose | 5~8% |
| Colloidal silica | 1~1.5% |
| Magnesium stearate | 0.3~0.8%. |

5. The pharmaceutical composition as claimed in any one of claims 1-4, wherein the allopregnanolone derivative is selected from a crystal form I of compound (II); the crystal form I has an X-ray powder diffraction pattern comprising absorption peaks at the following 2θ angles:
| No. | 2θ±0.2° | d value | Relative intensity | No. | 2θ±0.2° | d value | Relative intensity |
|---|---|---|---|---|---|---|---|
| 1 | 6.27 | 14.08 | 14.1% | 7 | 15.12 | 5.85 | 74.3% |
| 2 | 10.90 | 8.11 | 31.8% | 8 | 15.77 | 5.61 | 36.5% |
| 3 | 12.02 | 7.35 | 4.90% | 9 | 17.35 | 5.11 | 24.8% |
| 4 | 13.27 | 6.67 | 54.0% | 10 | 17.78 | 4.98 | 100.0% |
| 5 | 13.49 | 6.56 | 62.2% | 11 | 20.86 | 4.26 | 14.0% |
| 6 | 14.24 | 6.22 | 42.7% | 12 | 23.95 | 3.71 | 16.2% |

6. An immediate-release solid formulation, comprising the pharmaceutical composition as claimed in any one of claims 1-5, wherein:
preferably, the immediate-release solid formulation is a capsule, a granule, or a tablet.

7. A method for preparing the immediate-release solid formulation as claimed in claim 6 using the pharmaceutical composition as claimed in any one of claims 1-5, comprising the following steps:
1) sieving: sieving the active ingredient compound (I) and excipients separately to obtain sieved materials;
2) premixing: thoroughly mixing the sieved compound (I), fillers other than mannitol, the glidant, the binder, an optional disintegrant, and optionally part of mannitol to obtain a premixed material;
3) granulation: granulating the premixed material to obtain a granulated material;
4) final mixing: thoroughly mixing the granulated material with the lubricant and all or part of mannitol to obtain a final mixture; and
5) capsule filling: filling capsule shells with the final mixture.

8. The method as claimed in claim 7, wherein in step 3), the granulation is dry granulation; in step 4), the amount of mannitol added is less than 15% by weight of the pharmaceutical composition.

9. The method as claimed in claim 7 or 8, wherein in step 3), the granulation is dry granulation; in step 4), the amount of mannitol added accounts for 5-10% by weight of the pharmaceutical composition.

10. Use of the pharmaceutical composition as claimed in any one of claims 1-5 or the immediate-release solid formulation as claimed in claim 6 for the manufacturing of a medicament for the prevention or treatment of central nervous system-related diseases, or for the manufacturing of a medicament for sedation and hypnosis, wherein the central nervous system-related diseases are, for example, Alzheimer's disease, epilepsy, and depression, particularly postpartum depression, traumatic brain injury, or essential tremor.
